# EUROPÄISCHE PATENTSCHRIFT

(11) **EP 3 315 496 B1**
(45) Veröffentlichungstag und Bekanntmachung des Hinweises auf die Patenterteilung: **15.05.2019**
(21) Anmeldenummer: 17173459.3
(22) Anmeldetag: 30.05.2017
(51) Int. Cl.: C07D 303/48, C07F 7/18, C08C 19/25, C08K 5/5435, B60C 1/00, C07F 7/02, C08K 5/5415, C08L 9/00

(54) **3-GLYCIDYLOXYPROPYLTRIALKOXYSILANE MIT LANGKETTIGEN ALKOXYGRUPPEN, VERFAHREN ZUR HERSTELLUNG UND VERWENDUNG**
3-GLYCIDYLOXYPROPYLTRIALKOXYSILANES WITH LONG-CHAIN ALKOXY GROUPS, METHOD FOR PREPARATION AND USE
3-GLYCIDYLOXYPROPYLTRIALKOXYSILANE AYANT DES GROUPES ALKOXY À LONGUES CHAÎNES ET PROCÉDÉ DE FABRICATION ET D'UTILISATION

(30) Priorität: 25.10.2016 EP 16195474
(43) Veröffentlichungstag der Anmeldung: 02.05.2018
(73) Patentinhaber: Evonik Degussa GmbH, 45128 Essen (DE)
(72) Erfinder: Krafczyk, Roland, 79618 Rheinfelden (DE); Köpfer, Alexander, 79669 Zell im Wiesental (DE); Rosenstingl, Sebastian, 79618 Rheinfelden (DE)

(56) Entgegenhaltungen:
- EP-A2- 0 773 231
- DE-A1- 3 314 552
- JP-A- S62 199 612
- PLUEDDEMANN E P ET AL: "Epoxyorganosiloxanes", JOURNAL OF THE AMERICAN CHEMICAL SOCIETY, THE SOCIETY, Bd. 81, 5. Juni 1959 (1959-06-05), Seiten 2632-2635, XP007903953, ISSN: 0002-7863, DOI: 10.1021/JA01520A008

## Beschreibung

Die vorliegende Erfindung betrifft neue 3-Glycidyloxypropyltrialkoxysilane mit ausgewählten langkettigen Alkoxyresten, ein spezielles Verfahren zu deren Herstellung sowie deren Verwendung.

In Plueddemann et al, JOURNAL OF THE AMERICAN CHEMICAL SOCIETY, THE SOCIETY, Bd. 81,5. Juni 1959 (1959-06-05), Seiten 2632-2635, wird ein Verfahren zur Herstellung eines Epoxyorganosilans durch Epoxidierung von olefinischen Organosilan-Verbindungen mit Peressigsäure oder durch die Reaktion zwischen Siliciumhydriden und ungesättigten Epoxidverbindungen offenbart.

In EP 0773231 werden Polymere beschrieben, die mit Hilfe von 3-Glycidyloxypropyltrialkoxysilanen, funktionalisiert wurden. Werden diese Polymere in Mischungen für Reifenlaufflächen eingesetzt, kann der Rollwiderstand von Reifen reduziert werden. Diese funktionalisierten Polymere werden hergestellt, indem man zum Kettenabbruch der anionischen Polymerisation 3-Glycidyloxypropyltrialkoxysilane einsetzt. Bei der Reaktion wird u.a. der entsprechende Alkohol freigesetzt. Setzt man, wie in EP 0773231 beschrieben, das Produkt 3-Glycidyloxypropyltrimethoxysilan (Dynasylan® GLYMO) ein, so wird entsprechend Methanol freigesetzt. Da anionische Polymerisationen in einem inerten Lösungsmittel, wie beispielsweise n-Hexan oder Cyclohexan durchgeführt werden, so ist der beim Kettenabbruch freiwerdende Alkohol vom Lösungsmittel abzutrennen, da beim Wiedereinsatz des Lösungsmittels der Alkohol ebenfalls die Polymerisation abbrechen würde. Ferner besitzt Methanol einen ähnlichen Siedepunkt wie das Lösungsmittel n-Hexan oder Cyclohexan und kann somit nur sehr schwer bzw. nur mit einem hohen destillativen Aufwand abgetrennt werden, um das Lösemittel wieder nutzbar zu machen.

Umesterungsreaktionen als solche sind wohl bekannt, u.a. bei Alkoxysilanen. Meist wird eine Säure als Umesterungskatalysator eingesetzt.

DE-AS-1010739 lehrt die Herstellung von Polykieselsäureestern durch Umsetzung von Tetraethoxysilan mit höheren Alkoholen wie Cyclohexanol, Methylcyclohexanol sowie Phenolen in Gegenwart einer wasserfreien oder wässrigen Carbonsäure als Kondensationsmittel, beispielsweise Essig- oder Ameisensäure. Die bei der Umsetzung entstehenden flüchtigen Verbindungen, wie niederer Alkohol, wurden abdestilliert. Das Produkt ist säurehaltig.

US 2846459 offenbart die Herstellung bromierter Alkylsilikate durch Umesterung, wobei in den Beispielen von Polykieselsäureethylester ausgegangen und dieser u.a. mit einem Gemisch aus 2,3-Dibrompropan-1-ol und 2-Ethylhexanol umgesetzt wurde. Dabei kamen Natriummethanolat bzw. ein Gemisch aus Natriummethanolat und Kaliumcarbonat als Katalysator zum Einsatz. Auch hier wurden die nach der Umsetzung noch leicht flüchtigen Verbindungen, wie niederer Alkohol, abdestilliert und so entsprechende bromierte Alkylsilikate erhalten.

Aus Beispiel 7 der EP1035184A1 ist die Umsetzung von 100 g Ethylsilikat mit 18 g 2-Ethylhexanol in Gegenwart von Schwefelsäure als Katalysator zu entnehmen. Die Umsetzung erfolgte über 1 Stunde bei 120 °C. Die danach noch vorliegenden leicht flüchtigen Bestandteile wurden abdestilliert. Dabei wurde ein Alkylsilikat erhalten, das noch einen Gehalt von 94 Mol-% Ethyl und ein Molekulargewichtgewicht von 1750 g/mol aufwies. Hier erfolgte nur eine Teilumesterung mit geringer Ausbeute und das Produkt ist darüber hinaus aufgrund der eingesetzten und im Produkt verbleibenden Restmenge an Schwefelsäure sauer. Die Weiterverarbeitung des Produkts erfolgte in THF.

In JP S62 199612 A ist die Verbindung 2-[[3-[tris(pentyloxy)silyl]propoxy]metyl]]-Oxiran genannt.

Ferner ist der DE 33 14 552 A1 das 2-[[3-[tris(pentyloxy)silyl]propoxy]metyl]]-Oxiran zu entnehmen.

3-Glycidyloxypropyltrialkoxysilane mit langkettigen Alkoxyresten als solche sind somit nahezu unbeschrieben.

Die Glycidyloxy-Gruppe in 3-Glycidyloxypropyltrimethoxysilan bzw. 3-Glycidyloxypropyltriethoxysilan stellt eine sehr reaktive Gruppe dar, beispielsweise gegenüber nukleophilen Verbindungen und insbesondere in Gegenwart einer Säure.

Der vorliegenden Erfindung lag daher die Aufgabe zugrunde, 3-Glycidyloxypropyltrialkoxysilane mit langkettigen Alkoxyresten bzw. Zusammensetzungen, die einen hohen Anteil an 3-Glycidyloxypropyltrialkoxysilan mit langkettigen Alkoxyresten enthalten, einschließlich eines geeigneten Herstellverfahrens sowie ggf. auch eine geeignete Applikationsmöglichkeit für entsprechende Produkte bereitzustellen. Insbesondere bestanden die Anliegen, einen nicht sauren Katalysator für eine Umesterung aufzufinden und dabei eine möglichst hohe Produktausbeute unter Erhalt der Glycidyloxy-Gruppe zu erzielen. Weiter wurde angestrebt, die zuvor genannten Nachteile nach Möglichkeit zu vermeiden, zumindest aber zu mindern.

Die gestellte Aufgabe wird erfindungsgemäß entsprechend den Merkmalen in den hier vorliegenden Ansprüchen vorteilhaft gelöst.

Zusätzlich wird ausdrücklich darauf hingewiesen, dass alle der nachfolgend aufgeführten Merkmale, Vorzugsbereiche und Querverweise auf weitere Dokumente jeweils miteinander kombinierbar und somit umfänglich offenbart sind sowie der vorliegen Offenbarung zuzurechnen sind.

So wurde in überraschender Weise ein spezielles Verfahren zur Herstellung von 3-Glycidyloxypropyltrialkoxysilane mit langkettigen Alkoxyresten, d.h. solche mit mehr als 4 C-Atomen in der Kohlenstoffkette der Alkoxygruppe, insbesondere von 3-Glycidyloxypropyltri(-2-ethylhexoxy)silan und 3-Glycidyloxypropyltri(-2-propylheptoxy)silan, gefunden, indem man 3-Glycidyloxypropyl-trimethoxysilan oder 3-Glycidyloxypropyltriethoxysilan, eine definierte Menge an einem C5- bis C16-Alkohol, vorzugsweise C6- bis C12-Alkohol, besonders vorzugsweise C7- bis C11-Alkohol, ganz besonders vorzugsweise aus der Reihe der linearen oder verzweigten C8-, C9- oder C10-Alkohole, bevorzugt auch solche mit einer OH-Gruppe in ω-Stellung, insbesondere 2-Ethylhexanol oder 2-Propylheptanol, und eine definierte katalytische Menge an Titantetrabutanolat zusammenbringt, das Eduktgemisch unter Durchmischung auf eine Temperatur von ≤ 225 °C bei 1 bar (Umgebungsdruck), vorzugsweise auf eine Temperatur von 100 - 220 °C, aufheizt, reagieren lässt, d.h. umsetzt und im Anschluss an die Umsetzung Methanol bzw. Ethanol und gegebenenfalls vorliegenden überschüssigen langkettigen Alkohol, wie 2-Ethylhexanol bzw. 2-Propylheptanol, durch Destillation, vorzugsweise durch fraktionierte Destillation unter vermindertem Druck, aus dem so erhaltenen Produktgemisch entfernt und man das Zielprodukt bzw. die erfindungsgemäße Produktzusammensetzung als Sumpfprodukt nach Destillation in hervorragender Ausbeute gewinnt. Darüber hinaus ist die erfindungsgemäße Zusammensetzung nicht sauer und enthält vorteilhaft einen Gehalt an einer erfindungsgemäßen Zielverbindung, insbesondere 3-Glycidyloxypropyltri(-2-ethylhexoxy)silan bzw. 3-Glycidyloxypropyltri(-2-propylheptoxy)silan, von ≥ 90 Gew.-% sowie einen Gehalt an freiem Methanol bzw. Ethanol von ≤ 1 Gew.-%, jeweils bezogen auf das Produkt bzw. die Produktzusammensetzung.

Vorteilhaft können die hergestellte neuen 3-Glycidyloxypropyltrialkoxysilane mit langkettigen Alkoxyresten insbesondere 3-Glycidyloxypropyltri(-2-ethylhexoxy)silan bzw. 3-Glycidyloxypropyltri(-2-propylheptoxy)silan, zur endständigen Funktionalisierung von Lösungs-Styrol-Butadien-Kautschuk (S-SBR) sowie Butadien-Kautschuk (BR) während der anionischen Polymerisation eingesetzt werden und lassen sich anschließend leicht von dem dabei eingesetzten Lösungsmittel, wie n-Hexan oder Cyclohexan, abtrennen, so dass das Lösungsmittel wiederverwendet werden kann. Darüber hinaus zeichnen sich Gummimischungen für Reifenlaufflächen, die einen mit 3-Glycidyloxypropyltrialkoxysilanen mit langkettigen Alkoxyresten funktionalisierten Kautschuk enthalten, besonders vorteilhaft durch einen reduzieren den Rollwiderstand der Reifen aus.

Gegenstand der vorliegenden Erfindung sind somit die Verbindung 3-Glycidyloxypropyltri(-2-propylheptoxy)silan als solche.

Ferner ist Gegenstand der vorliegenden Erfindung ein Verfahren zur Herstellung eines 3-Glycidyloxypropyltrialkoxysilans mit langkettigen Alkoxygruppen
der allgemeinen Formel (I) 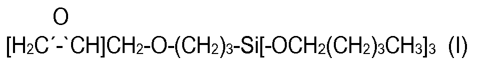 oder
der allgemeinen Formel (II) 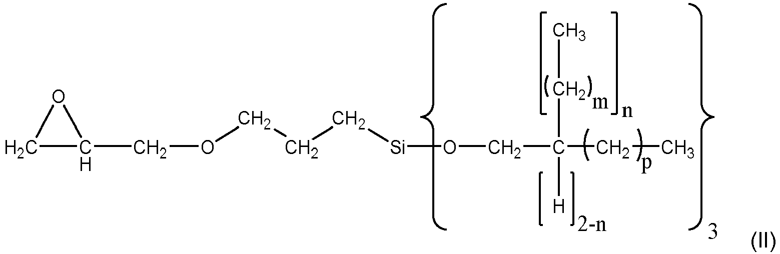 mit m= 1 oder 2, n= 0 oder 1, p=3, 4, 5, 6, 7, 8, 9 oder 10, insbesondere 3-Glycidyloxypropyltri(-2-ethylhexoxy)silan sowie 3-Glycidyloxypropyltri(-2-propylheptoxy)silan,
indem man
- 3-Glycidyloxypropyltrimethoxysilan oder 3-Glycidyloxypropyltriethoxysilan mit einer stöchiometrischen oder im Überschuss eingesetzten Menge eines längerkettigen Alkohols aus der Reihe der C5- bis C16-Alkohole,
- in Gegenwart von Titantetrabutanolat als Katalysator
- unter Durchmischung auf eine Temperatur kleiner gleich 225 °C erhitzt,
- reagieren lässt,
- im Anschluss an die Umsetzung Methanol bzw. Ethanol und überschüssigen Eduktalkohol durch Destillation, vorzugsweise unter vermindertem Druck, aus dem Produktgemisch entfernt und man das Produkt gewinnt.

Die allgemeine Formel (II) kann optional auch wie folgt dargestellt werden:

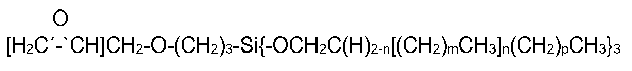

Vorzugsweise setzt man beim erfindungsgemäßen Verfahren als langkettigen Alkohol 2-Ethylhexan-1-ol, Isononanol oder2-Propylheptan-1-ol ein, wobei Isononanol, auch Isononan-1-ol bzw. 1-Isononanol genannt, im Wesentlichen für seine Isomeren oder Isomerengemische steht, u.a. 3,5,5-Trimethyl-1-hexanol, isomere Dimethyl-1-heptanole, 7-Methyloctan-1-ol, um nur einige zu nennen.

Vorteilhaft setzt man beim erfindungsgemäßen Verfahren 3-Glycidyloxypropyltrimethoxysilan oder 3-Glycidyloxypropyltriethoxysilan und langkettigen Alkohol, insbesondere 2-Ethylhexanol sowie 2-Propylheptanol, in einem molaren Verhältnis von 1 : 3 bis 6, bevorzugt 1 : 3 bis 5, besonders bevorzugt 1 : 3,0 bis 4, insbesondere 1 : 3,0 bis 3,9 ein. Werden Reinheiten >95% benötigt, kann der langkettige Alkohol vorteilhaft stöchiometrisch im molaren Verhältnis 3-Glycidyloxypropyltrimethoxysilan oder 3-Glycidyloxypropyltriethoxysilan zu langkettigen Alkohol von 1 : 3,0 eingesetzt werden.

Ferner setzt man beim erfindungsgemäßen Verfahren vorteilhaft 0,01 bis 0,5 Gew.-%, bevorzugt von 0,05 bis 0,2 Gew.-%, besonders bevorzugt 0,1 Gew.-%, Titantetrabutanolat, bezogen auf die eingesetzte Menge an 3-Glycidyloxypropyltrimethoxysilan bzw. Glycidyloxypropyltriethoxysilan, ein.

Geeigneterweise führt man beim erfindungsgemäßen Verfahren die Umsetzung bei einer Temperatur von 100 bis 220 °C, bevorzugt von 120 bis 150 °C, besonders bevorzugt von 120 bis 140 °C, und über einen Zeitraum von 6 bis 24 Stunden, bevorzugt von 9 bis 18 Stunden durch.

Im Allgemeinen führt man das erfindungsgemäße Verfahren wie folgt durch:
In der Regel legt man ein Gemisch beispielsweise aus 3-Glycidyloxypropyltrimethoxysilan und einer stöchiometrischen bis molgewichtsmäßig überschüssigen Menge an 2-Ethylhexanol oder 2-Propylheptanol sowie einer katalytischen Menge an Titantetrabutanolat in einer geeigneten Reaktionsapparatur -zum Beispiel auf Basis Reaktionsgefäß mit Zuführungen zur Eduktdosierung, Rührer, Heizung, Temperatur-kontrolle/-regelung, Rückflusskühler und Brücke mit Vorlage- vor, erhitzt das Gemisch unter Rühren bevorzugt auf eine Temperatur von 100 bis 220 °C, insbesondere auf eine Temperatur im Bereich von 120 bis 140 °C, lässt das Gemisch bei der Temperatur hinreichend lange reagieren bzw. umsetzen, vorzugsweise über 6 bis 24 Stunden, und destilliert geeigneterweise im Anschluss an die Umsetzungsphase die im so erhaltenen Reaktions- bzw. Produktgemisch noch vorliegenden leicht flüchten Komponenten, wie Methanol bzw. Ethanol, sowie den ggf. vorliegenden überschüssigen langkettigen Alkohol, wie 2-Ethylhexanol bzw. 2-Propylheptanol, geeigneterweise unter vermindertem Druck, ab, um das Reaktions- bzw. Produktgemisch mittels Destillation aufzuarbeiten und so das Produkt bzw. die Produktzusammensetzung zu gewinnen. Beispielsweise kann man zur Durchführung der Destillation das nach Umsetzung vorliegende Reaktions- bzw. Produktgemisch aus dem Reaktionsgefäß in eine gesonderte Destillationseinheit überführen und mittels fraktionierter Destillation aufarbeiten. Darüber hinaus kann man bei der Destillation Vakuum anlegen, das heißt, unter vermindertem Druck destillieren und fakultativ zusätzlich Stickstoff durch das im Sumpf der Destillationsapparatur befindliche Produkt bzw. Produktgemisch leiten. Das Produkt bzw. die erfindungsgemäße Zusammensetzung gewinnt man somit vorteilhaft als farblos bis gelbliche, leicht viskose Flüssigkeit im Sumpf der verwendeten Destillationsapparatur. Alternativ lassen sich die flüchtigen Alkohole, wie Methanol bzw. Ethanol sowie die überschüssigen langkettigen Alkohole, mit Hilfe eines Dünnschichtverdampfers unter Vakuum schonend vom Produkt bzw. Produktgemisch abtrennen, welches vorteilhaft als sogenannter "Schwersieder" gesammelt wird.

Überraschend erzielt man bei der Durchführung des erfindungsgemäßen Verfahrens in besonders vorteilhafter Weise eine nahezu vollständige Umesterung mit einer Ausbeute von≥90%, insbesondere ≥ 95 %, und ist somit in die günstige Lage versetzt, ein entsprechendes neues Reaktionsprodukt bereitstellen zu können. So kann man nach dem erfindungsgemäßen Verfahren vorteilhaft neue 3-Glycidyloxypropyltrialkoxysilane mit langkettigen Alkoxygruppen gemäß Formel (I) und Formel (II) sowie entsprechende Zusammensetzungen mit einem hohen Gehalt an entsprechenden 3-Glycidyloxypropyltrialkoxysilane mit langkettigen Alkoxygruppen, insbesondere an 3-Glycidyloxypropyltri(-2-ethylhexoxy)silan und 3-Glycidyloxypropyltri(-2-propylheptoxy)silan. von ≥ 90 Gew.-%, bevorzugt ≥ 95 Gew.-%, erhalten.

Gegenstand der vorliegenden Erfindung sind daher auch Zusammensetzungen mit einem Gehalt an 3-Glycidyloxypropyltri(-2-ethylhexoxy)silan bzw. 3-Glycidyloxypropyltri(-2-propylheptoxy)silan von ≥ 90 Gew.-%, bevorzugt ≥ 95 Gew.-%, die gemäß dem erfindungsgemäßen Verfahren erhältlich sind, wobei die Komponenten in der Zusammensetzung in Summe 100 Gew.-% ergeben.

Weiter ist Gegenstand der Erfindung eine Zusammensetzung bzw. erfindungsgemäß hergestellte Zusammensetzung mit einem Gehalt an 3-Glycidyloxypropyltri(-2-ethylhexoxy)silan bzw. 3-Glycidyloxypropyltri(-2-propylheptoxy)silan von≥90 Gew.-% bevorzugt ≥ 95 Gew.-%, bezogen auf die Zusammensetzung.

Ferner weist eine erfindungsgemäße Zusammensetzung bzw. erfindungsgemäß hergestellte Zusammensetzung bevorzugt einen Gehalt an Methanol bzw. Ethanol von ≤ 1 Gew.-%, bevorzugt ≤ 0,5 Gew.-% bis hin zur Nachweisgrenze, bezogen auf die Zusammensetzung, auf und zeichnet sich somit auch aus ökologischer Sicht zusätzlich durch einen nur sehr geringen Anteil an VOC (Volatile Organic Compounds = flüchtige organische Verbindungen) aus. Auch können erfindungsgemäße Zusammensetzungen bzw. erfindungsgemäß hergestellte Zusammensetzungen einen Gehalt an so genannten Mischestern enthalten, bevorzugt -aber nicht ausschließlich- solche aus der Reihe 3-Glycidyloxypropyldi(-2-ethylhexoxy)monomethoxysilan, 3-Glycidyloxypropyldi(-2-ethylhexoxy)monoethoxysilan, 3-Glycidyloxypropylmono(-2-ethylhexoxy)dimethoxysilan sowie 3-Glycidyloxypropylmono(-2-ethylhexoxy)diethoxysilan, geeigneterweise mit einem Gehalt an Mischester von ≤10 Gew.-%, vorzugsweise ≤5 Gew.-%, wobei alle Komponenten in einer erfindungsgemäßen Zusammensetzung bzw. erfindungsgemäß hergestellten Zusammensetzungen in Summe 100 Gew.-% ergeben.

Darüber hinaus ist Gegenstand der vorliegenden Erfindung die Verwendung mindestens eines der erfindungsgemäßen 3-Glycidyloxypropyltrialkoxysilane mit langkettigen Alkoxygruppen gemäß Formel (I) und Formel (II), insbesondere Glycidyloxypropyltri(-2-ethylhexoxy)silan oder 3-Glycidyloxypropyltri(-2-propylheptoxy)silan, zur Funktionalisierung von Kautschuk, wobei das 3-Glycidyloxypropyl-trialkoxysilane mit langkettigen Alkoxygruppen, insbeosndere 3-Glycidyloxypropyltri(-2-ethylhexoxy)silan oder 3-Glycidyloxypropyltri(-2-propylheptoxy)silan, zum Kettenabbruch bei einer anionischen Polymerisation bevorzugt eingesetzt wird. Bevorzugt verwendet man mindestens eines der besagten 3-Glycidyloxypropyltrialkoxysilane mit langkettigen Alkoxygruppen zur Funktionalisierung bzw. Modifizierung von Kautschuk.

Ferner verwendet man vorteilhaft einen mit mindestens einem 3-Glycidyloxypropyltrialkoxysilan mit langkettigen Alkoxygruppen der Formel (I) sowie der Formel (II), vorzugsweise 3-Glycidyloxypropyltri(-2-ethylhexoxy)silan oder. 3-Glycidyloxypropyltri(-2-propylheptoxy)silan, modifizierten Kautschuk in Gummimischungen; insbesondere eines mit 3-Glycidyloxypropyltri(-2-ethylhexoxy)silan oder 3-Glycidyloxypropyltri(-2-propylheptoxy)silan modifizierten S-SBR oder BR-Kautschuks in Gummimischungen für Laufstreifen in Reifen.

So können die neuen erfindungsgemäßen Verbindungen bzw. Zusammensetzungen zum Beispiel - aber nicht ausschließlich- als Kopplungsreagenz bei der Herstellung von funktionellen Polymeren, wie Butadienkautschuk oder bei Lösungs-Styrol-Butadien-Kautschuk, vorteilhaft eingesetzt werden.

Die vorliegende Erfindung wird durch die nachfolgenden Beispiele näher erläutert, ohne den Gegenstand der Erfindung zu beschränken:

### Beispiele:

### Verwendete Chemikalien:

Dynasylan® GLYMO (3-Glycidyloxypropyltrimethoxysilan), Evonik Resource Efficiency GmbH Titantetrabutanolat, Sigma-Aldrich
2-Ethylhexanol, Sigma-Aldrich
2-Propylheptanol, Evonik Performance Materials GmbH

### Analytische Methoden:

### NMR-Messungen:

Instrument: Bruker
Frequenz: 100,6 MHz (¹³C-NMR)
Scans: 1024 (¹³C-NMR)
Temperatur: 296 K
Lösungsmittel: CDCl₃
Standard: Tetramethylsilan

### Gaschromatographische Bestimmung von Alkohol:

Alle Angaben verstehen sich als Richtwerte. Säulen von ähnlicher Polarität z.B. von anderen Herstellern sind zugelassen. Ist die Trennung nachweislich auch mit einem Gerät mit gepackter Säule erreichbar, ist auch dieses zugelassen.

Bei der Handhabung der Proben ist deren Feuchtigkeitsempfindlichkeit zu beachten.

| | | |
|---|---|---|
| Gerät: | Kapillar-Gaschromatograph mit WLD und Integrator z.B. HP 5890 mit Integrator HP 3396 | |
| Trennsäule: | Kapillarsäule | |
| | Länge: | 25 m |
| | innerer Durchmesser: | 0,20 mm |
| | Filmdicke: | 0,33 mm |
| | stationäre Phase: | HP Ultra 1 |
| Temperaturen: | Säulenofen: | 120 °C - 2 min - 10 °/min - 275 °C - 8 min |
| | Injektor: | 250 °C |
| | Detektor: | 280 °C |
| Trägergas: | Helium | |
| | Strömung: | ca. 1 ml/min |
| | Split-Verhältnis: | ca. 1:100 |
| Injizierte Probe: | 0,4 ml | |

Auswertung erfolgt durch Normierung auf 100 Flächen-%.

### Beispiel 1:

Dynasylan® GLYMO (23,6 g, 100 mmol, 1,0 eq.), 2-Ethylhexanol (39,1 g, 300 mmol, 3,0 eq.) und Ti(O*n*Bu)₄ (23,6 mg, 0,1 Gew.-% bzgl. Dynasylan® GLYMO) wurden vorgelegt und 12 h auf 130 °C erhitzt. Dann wurde bei 130 °C und 0,1 mbar das Produkt von flüchtigen Bestandteilen getrennt. Als Umsetzungsprodukt (56,1 g) wurde eine leicht gelbliche, leicht viskose Flüssigkeit erhalten. Das Umsetzungsprodukt wurde mittels ¹³C-NMR analysiert. Die Analyse belegt, dass als Umsetzungsprodukt ein 3-Glycidyloxypropyltri(-2-ethylhexoxy)silan erhalten wurde.

¹³C-NMR (100 MHz, CDCl₃): δ = 74.0 (s, 1C), 71.5 (s, 1C), 64.9 (s, 3C), 50.9 (s, 1C), 44.4 (s, 1C), 41.9 (s, 3C), 30.2 (s, 3C), 29.2 (s, 3C), 23.5 (s, 3C), 23.2 (s, 3C), 14.1 (s, 3C), 11.2 (s, 3C), 6.4 (s, 1C) ppm.

Die Umesterungsausbeute betrug 95%, d. h. 95% der Methoxy-Gruppen des eingesetzten Dynasylan® GLYMO wurden erfindungsgemäß durch 2-Ethylhexoxy-Gruppen ersetzt bzw. umgeestert.

### Beispiel 2:

Dynasylan® GLYMO (23,6 g, 100 mmol, 1,0 eq.), 2-Ethylhexanol (50,1 g, 390 mmol, 3,9 eq.) und Ti(O*n*Bu)₄ (23,6 mg, 0,1 Gew.-% bzgl. Dynasylan® GLYMO) wurden vorgelegt und 16 h auf 130 °C erhitzt. Dann wurde bei 130 °C und 0,1 mbar das Produkt von flüchtigen Bestandteilen getrennt. Als Umsetzungsprodukt (57,9 g) wurde eine leicht gelbliche, leicht viskose Flüssigkeit erhalten. Das Umsetzungsprodukt wurde mittels ¹³C-NMR analysiert. Die Analyse belegt, dass als Umsetzungsprodukt ein 3-Glycidyloxypropyltri(-2-ethylhexoxy)silan erhalten wurde.

¹³C-NMR (100 MHz, CDCl₃): δ = 74.0 (s, 1C), 71.5 (s, 1C), 64.9 (s, 3C), 50.9 (s, 1C), 44.4 (s, 1C), 41.9 (s, 3C), 30.2 (s, 3C), 29.2 (s, 3C), 23.5 (s, 3C), 23.2 (s, 3C), 14.1 (s, 3C), 11.2 (s, 3C), 6.4 (s, 1C) ppm.

Die Umesterungsausbeute betrug 98%, d. h. 98% der Methoxy-Gruppen des eingesetzten Dynasylan® GLYMO wurden erfindungsgemäß durch 2-Ethylhexoxy-Gruppen ersetzt bzw. umgeestert.

### Beispiel 3:

Dynasylan® GLYMO (23,6 g, 100 mmol, 1,0 eq.), 2-Propylheptanol (47,5 g, 300 mmol, 3,0 eq.) und Ti(O*n*Bu)₄ (23,6 mg, 0,1 Gew.-% bzgl. Dynasylan® GLYMO) wurden vorgelegt und 12 h auf 130 °C erhitzt. Dann wurde bei 130 °C und 0,1 mbar das Produkt von flüchtigen Bestandteilen getrennt. Als Umsetzungsprodukt (59,0 g) wurde eine leicht gelbliche, leicht viskose Flüssigkeit erhalten. Das Umsetzungsprodukt wurde mittels ¹³C-NMR analysiert. Die Analyse belegt, dass als Umsetzungsprodukt ein 3-Glycidyloxypropyltri(-2-propylheptoxy)silan erhalten wurde.

¹³C-NMR (100 MHz, CDCl₃): δ = 73.9 (s, 1C), 71.4 (s, 1C), 65.3 (s, 3C), 50.9 (s, 1C), 44.3 (s, 1C), 40.2 (s, 3C), 33.4 (s, 3C), 32.5 (s, 3C), 31.0 (s, 3C), 26.6 (s, 3C), 22.8 (s, 3C), 20.1 (s, 3C), 14.6 (s, 3C), 14.2 (s, 3C), 6.4 (s, 1C) ppm.

Die Umesterungsausbeute betrug 96 %, d. h. 96 % der Methoxy-Gruppen des eingesetzten Dynasylan® GLYMO wurden erfindungsgemäß durch 2-Propylheptoxy-Gruppen ersetzt bzw. umgeestert.

### Beispiel 4:

Dynasylan® GLYMO (23,6 g, 100 mmol, 1,0 eq.), 2-Propylheptanol (61,7 g, 390 mmol, 3,9 eq.) und Ti(O*n*Bu)₄ (23,6 mg, 0,1 Gew.-% bzgl. Dynasylan® GLYMO) wurden vorgelegt und 18 h auf 130 °C erhitzt. Dann wurde bei 130 °C und 0,1 mbar das Produkt von flüchtigen Bestandteilen getrennt. Als Umsetzungsprodukt (60,9 g) wurde eine leicht gelbliche, leicht viskose Flüssigkeit erhalten. Das Umsetzungsprodukt wurde mittels ¹³C-NMR analysiert. Die Analyse belegt, dass als Umsetzungsprodukt ein 3-Glycidyloxypropyltri(-2-propylheptoxy)silan erhalten wurde.

¹³C-NMR (100 MHz, CDCl₃): δ = 73.9 (s, 1C), 71.4 (s, 1C), 65.3 (s, 3C), 50.9 (s, 1C), 44.3 (s, 1C), 40.2 (s, 3C), 33.4 (s, 3C), 32.5 (s, 3C), 31.0 (s, 3C), 26.6 (s, 3C), 22.8 (s, 3C), 20.1 (s, 3C), 14.6 (s, 3C), 14.2 (s, 3C), 6.4 (s, 1C) ppm.

Die Umesterungsausbeute betrug 99 %, d. h. 99 % der Methoxy-Gruppen des eingesetzten Dynasylan® GLYMO wurden erfindungsgemäß durch 2-Propylheptoxy-Gruppen ersetzt bzw. umgeestert.

## Patentansprüche

1. 3-Glycidyloxypropyltri(-2-propylheptoxy)silan.

2. Verfahren zur Herstellung eines 3-Glycidyloxypropyltrialkoxysilans mit langkettigen Alkoxygruppen der allgemeinen Formel (I) 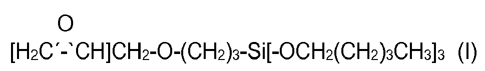 oder
der allgemeinen Formel (II) 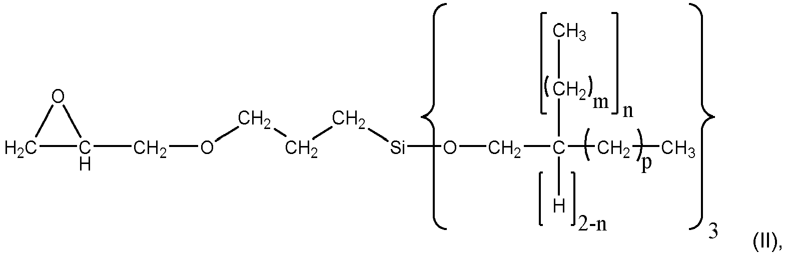 mit m= 1 oder 2, n= 0 oder 1, p=3, 4, 5, 6, 7, 8, 9 oder 10, insbesondere 3-Glycidyloxypropyltri(-2-ethylhexoxy)silan und 3-Glycidyloxypropyltri(-2-propylheptoxy)silan, indem man
- 3-Glycidyloxypropyltrimethoxysilan oder 3-Glycidyloxypropyltriethoxysilan mit einer stöchiometrischen oder im Überschuss eingesetzten Menge eines längerkettigen Alkohols aus der Reihe der C5- bis C16-Alkohole,
- in Gegenwart von Titantetrabutanolat als Katalysator
- unter Durchmischung auf eine Temperatur kleiner gleich 225 °C erhitzt,
- reagieren lässt,
- im Anschluss an die Umsetzung Methanol bzw. Ethanol und überschüssigen Eduktalkohol durch Destillation, vorzugsweise unter vermindertem Druck, aus dem Produktgemisch entfernt und man das Produkt gewinnt.

3. Verfahren nach Anspruch 2,
wobei man als langkettigen Alkohol 2-Ethylhexan-1-ol, Isononan-1-ol oder 2-Propylheptan-1-ol einsetzt.

4. Verfahren nach Anspruch 2 oder 3,
wobei man 3-Glycidyloxypropyltrimethoxysilan oder Glycidyloxypropyltriethoxysilan und einen langkettigen Alkohol, insbesondere 2-Ethylhexanol oder 2-Propylheptanol, in einem molaren Verhältnis von 1 : 3 bis 6, bevorzugt 1 : 3 bis 5, besonders bevorzugt 1 : 3,0 bis 4, insbesondere 1 : 3,0 bis 3,9, einsetzt.

5. Verfahren nach einem der vorangehenden Ansprüche,
wobei man 0,01 bis 0,5 Gew.-% Titantetrabutanolat, bezogen auf die eingesetzte Menge an 3-Glycidyloxypropyltrimethoxysilan, einsetzt, bevorzugt von 0,05 bis 0,2 Gew.-%, besonders bevorzugt 0,1 Gew.-%.

6. Verfahren nach einem der vorangehenden Ansprüche,
wobei man die Umsetzung bei einer Temperatur von 100 bis 220 °C durchführt, bevorzugt von 120 bis 150 °C, besonders bevorzugt von 120-140 °C.

7. Verfahren nach einem der vorangehenden Ansprüche,
wobei man die Umsetzung über 6 bis 24 Stunden durchführt, bevorzugt über 9 bis 18 Stunden.

8. Verfahren nach einem der vorangehenden Ansprüche,
wobei man die Umesterung mit einer Ausbeute von≥90%, vorzugsweise ≥ 95 %, durchführt.

9. Verfahren nach einem der vorangehenden Ansprüche,
wobei man ein Produkt als Zusammensetzung mit einem Gehalt an 3-Glycidyloxypropyltri(-2-ethylhexoxy)silan bzw. 3-Glycidyloxypropyltri(-2-propylheptoxy)silan von≥90 Gew.-% erhält, bevorzugt ≥ 95 %, wobei die Komponenten in der Zusammensetzung in Summen 100 Gew.-% ergeben.

10. Zusammensetzung mit einem Gehalt an 3-Glycidyloxypropyltri(-2-ethylhexoxy)silan oder 3-Glycidyloxypropyltri(-2-propylheptoxy)silan von≥90 Gew.-% erhältlich nach einem der vorangehenden Ansprüche, wobei die Komponenten in der Zusammensetzung in Summen 100 Gew.-% ergeben.

11. Zusammensetzung nach Anspruch 10 mit einem Gehalt an 3-Glycidyloxypropyltri(-2-ethylhexoxy)silan oder 3-Glycidyloxypropyltri(-2-propylheptoxy)silan von≥90 Gew.-%, bevorzugt ≥ 95 %.

12. Zusammensetzung einem nach Anspruch 10 oder 11 mit einem Gehalt an Alkoholen von Methanol oder Ethanol von ≤ 1 Gew.-%, bevorzugt ≤ 0,5 Gew.-%, bezogen auf die Zusammensetzung.

13. Zusammensetzung nach einem der vorangehenden Ansprüche mit einem Gehalt an Mischestern ≤10 Gew.-%, vorzugsweise ≤5 Gew.-%, wobei solche Mischester vorzugsweise aus der Reihe 3-Glycidyloxypropyldi(-2-ethylhexoxy)monomethoxysilan, 3-Glycidyloxypropyldi(-2-ethylhexoxy)monoethoxysilan, 3-Glycidyloxypropylmono(-2-ethylhexoxy)dimethoxysilan sowie 3-Glycidyloxypropylmono(-2-ethylhexoxy)diethoxysilan ausgewählt sind.

14. Verwendung mindestens eines 3-Glycidyloxypropyltrialkoxysilans mit langkettigen Alkoxygruppen nach einem der vorangehenden Ansprüche zur Funktionalisierung von Kautschuk, wobei 3-Glycidyloxypropyltri(-2-ethylhexoxy)silan oder 3-Glycidyloxypropyltri(-2-propylheptoxy)silan zum Kettenabbruch bei einer anionischen Polymerisation bevorzugt eingesetzt wird.

15. Verwendung mindestens eines 3-Glycidyloxypropyltrialkoxysilans mit langkettigen Alkoxygruppen nach einem der vorangehenden Ansprüche zur Modifizierung von Kautschuk.

16. Verwendung eines mit mindestens einem 3-Glycidyloxypropyltrialkoxysilan mit langkettigen Alkoxygruppen, vorzugsweise 3-Glycidyloxypropyltri(-2-ethylhexoxy)silan oder. 3-Glycidyloxypropyltri(-2-propylheptoxy)silan, modifizierten Kautschuks nach einem der vorangehenden Ansprüche in Gummimischungen.

17. Verwendung eines mit 3-Glycidyloxypropyltri(-2-ethylhexoxy)silan oder 3-Glycidyloxypropyltri(-2-propylheptoxy)silan modifizierten S-SBR oder BR-Kautschuks in Gummimischungen nach einem der vorangehenden Ansprüche für Laufstreifen in Reifen.

## Claims

1. 3-Glycidyloxypropyltri(-2-propylheptoxy)silane.

2. Process for producing a 3-glycidyloxypropyltrialkoxysilane having long-chain alkoxy groups of general formula (I) 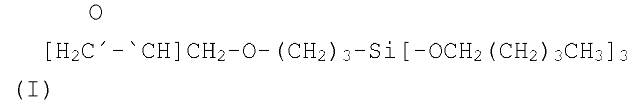 or
of general formula (II) 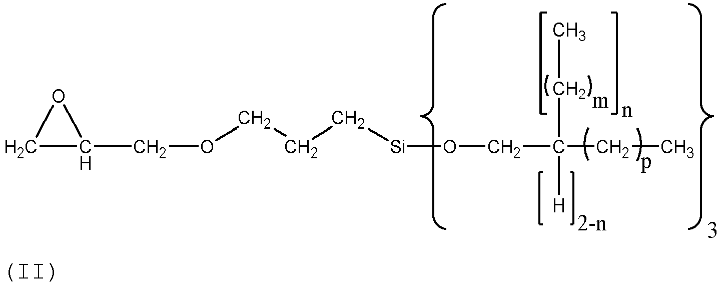 with m= 1 or 2, n= 0 or 1, p=3, 4, 5, 6, 7, 8, 9 or 10, in particular 3-glycidyloxypropyltri(-2-ethylhexoxy)silane and 3-glycidyloxypropyltri(-2-propylheptoxy) silane,
where
- 3-glycidyloxypropyltrimethoxysilane or 3-glycidyloxypropyltriethoxysilane, with a stoichiometric amount or an excess of a longer-chain alcohol from the group of the C5- to C16-alcohols,
- in the presence of titanium tetrabutoxide as catalyst,
- is heated with stirring to a temperature of not more than 225°C,
- reacted, and
- following the reaction, methanol/ethanol and excess reactant alcohol, are removed from the product mixture by distillation, preferably under reduced pressure, and the product is obtained.

3. Process according to Claim 2,
wherein the long-chain alcohol employed is 2-ethylhexan-1-ol, isononan-1-ol or 2-propylheptan-1-ol.

4. Process according to Claim 2 or 3,
wherein glycidyloxypropyltrimethoxysilane or glycidyloxypropyltriethoxysilane and a long-chain alcohol, in particular 2-ethylhexanol or 2-propylheptanol, are employed in a molar ratio of 1:3 to 6, preferably 1:3 to 5, particularly preferably 1:3.0 to 4, in particular 1:3.0 to 3.9.

5. Process according to any of the preceding claims, wherein 0.01% to 0.5% by weight of titanium tetrabutoxide based on the employed amount of 3-glycidyloxypropyltrimethoxysilane is employed, preferably from 0.05% to 0.2% by weight, particularly preferably 0.1% by weight.

6. Process according to any of the preceding claims, wherein the reaction is performed at a temperature of 100 to 220°C, preferably of 120 to 150°C, particularly preferably of 120-140°C.

7. Process according to any of the preceding claims, wherein the reaction is performed over a period of 6 to 24 hours, preferably of 9 to 18 hours.

8. Process according to any of the preceding claims, wherein the transesterification is performed with a yield of > 90%, preferably ≥ 95%.

9. Process according to any of the preceding claims, wherein a product is obtained as a composition having a content of 3-glycidyloxypropyltri(-2-ethylhexoxy)silane/3-glycidyloxypropyltri(-2-propylheptoxy)silane of ≥ 90% by weight, preferably > 95% wherein the components in the composition sum to 100% by weight.

10. Composition having a content of 3-glycidyloxypropyltri(-2-ethylhexoxy)silane or 3-glycidyloxypropyltri(-2-propylheptoxy)silane of > 90% by weight obtainable according to any one of the preceding claims, wherein the components in the composition sum to 100% by weight.

11. Composition according to Claim 10, having a content of 3-glycidyloxypropyltri(-2-ethylhexoxy)silane or 3-glycidyloxypropyltri(-2-propylheptoxy)silane of > 90% by weight, preferably > 95%.

12. Composition according to Claim 10 or 11, having a content of alcohols of methanol or ethanol of < 1% by weight, preferably < 0.5% by weight, based on the composition.

13. Composition according to any of the preceding claims, having a content of mixed esters < 10% by weight, preferably < 5% by weight, wherein such mixed esters are preferably selected from the group 3-glycidyloxypropyldi(-2-ethylhexoxy)monomethoxysilane, 3-glycidyloxypropyldi(-2-ethylhexoxy)monoethoxysilane, 3-glycidyloxypropylmono(-2-ethylhexoxy)dimethoxysilane and 3-glycidyloxypropylmono(-2-ethylhexoxy)diethoxysilane.

14. Use of at least one 3-glycidyloxypropyltrialkoxysilane having long-chain alkoxy groups according to any of the preceding claims for functionalization of rubber, wherein 3-glycidyloxypropyltri(-2-ethylhexoxy)silane or 3-glycidyloxypropyltri(-2-propylheptoxy)silane is preferably used for chain termination in an anionic polymerization.

15. Use of at least one 3-glycidyloxypropyltrialkoxysilane having long-chain alkoxy groups according to any of the preceding claims for modifying rubber.

16. Use of a rubber modified with at least one 3-glycidyloxypropyltrialkyloxysilane having long-chain alkoxy groups, preferably 3-glycidyloxypropyltri(-2-ethylhexoxy)silane or 3-glycidyloxypropyltri(-2-propylheptoxy)silane, according to any of the preceding claims in rubber mixtures.

17. Use of an S-SBR oder BR modified with 3-glycidyloxypropyltri(-2-ethylhexoxy)silane or 3-glycidyloxypropyltri(-2-propylheptoxy)silane in rubber mixtures according to any of the preceding claims for treads in tyres.

## Revendications

1. 3-glycidyloxypropyltri(-2-propylheptoxy)silane.

2. Procédé pour la préparation d'un 3-glycidyloxypropyltrialcoxysilane présentant des groupes alcoxy à longue chaîne de formule générale (I) 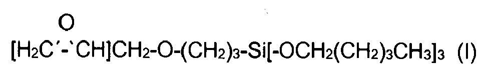 ou de formule générale (II) 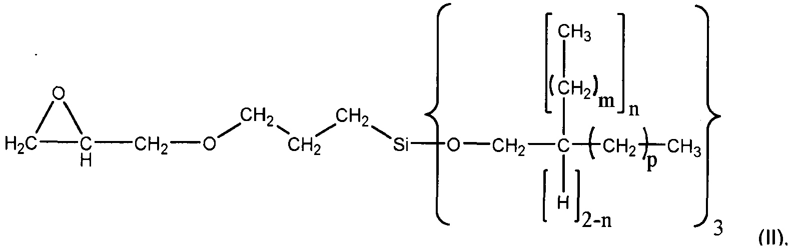 dans laquelle m = 1 ou 2, n = 0 ou 1, p = 3, 4, 5, 6, 7, 8, 9 ou 10, en particulier le 3-glycidyloxypropyltri(-2-éthylhexoxy)silane et le 3-glycidyloxypropyltri(-2-propylheptoxy)silane, en ce que
- on chauffe du 3-glycidyloxypropyltriméthoxysilane ou du 3-glycidyloxypropyltriéthoxysilane avec une quantité stoechiométrique ou en excès d'un alcool à longue chaîne de la série des alcools en C5-C16,
- en présence de tétrabutanolate de titane comme catalyseur
- sous agitation à une température inférieure à 225°C,
- on laisse réagir,
- après la réaction, on élimine le méthanol ou l'éthanol et l'alcool de départ en excès par distillation, de préférence sous pression réduite, à partir du mélange produit et on obtient le produit.

3. Procédé selon la revendication 2, dans lequel on utilise du 2-éthylhexan-1-ol, de l'isononan-1-ol ou du 2-propylheptan-1-ol comme alcool à longue chaîne.

4. Procédé selon la revendication 2 ou 3, dans lequel on utilise du 3-glycidyloxypropyltriméthoxysilane ou du glycidyloxypropyltriéthoxysilane et un alcool à longue chaîne, en particulier le 2-éthylhexanol ou le 2-propylheptanol, dans un rapport molaire de 1:3 à 6, de préférence de 1:3 à 5, de manière particulièrement préférée de 1:3,0 à 4, en particulier de 1:3,0 à 3,9.

5. Procédé selon l'une quelconque des revendications précédentes, dans lequel on utilise 0,01 à 0,5% en poids de tétrabutanolate de titane, de préférence 0,05 à 0,2% en poids, de manière particulièrement préférée 0,1 % en poids, par rapport à la quantité utilisée de 3-glycidyloxypropyltriméthoxysilane.

6. Procédé selon l'une quelconque des revendications précédentes, dans lequel on réalise la transformation à une température de 100 à 220°C, de préférence de 120 à 150°C, de manière particulièrement préférée de 120-140°C.

7. Procédé selon l'une quelconque des revendications précédentes, dans lequel on réalise la transformation sur 6 à 24 heures, de préférence sur 9 à 18 heures.

8. Procédé selon l'une quelconque des revendications précédentes, dans lequel on réalise la transestérification à un rendement ≥ 90%, de préférence ≥ 95%.

9. Procédé selon l'une quelconque des revendications précédentes, dans lequel on obtient un produit sous forme d'une composition, présentant une teneur en 3-glycidyloxypropyltri(-2-éthylhexoxy)silane ou en 3-glycidyloxypropyltri(-2-propylheptoxy)silane ≥ 90% en poids, de préférence ≥ 95%, la somme des composants dans la composition valant 100% en poids.

10. Composition présentant une teneur en 3-glycidyloxypropyltri(-2-éthylhexoxy)silane ou en 3-glycidyloxypropyltri(-2-propylheptoxy)silane ≥ 90% en poids, pouvant être obtenue selon l'une quelconque des revendications précédentes, la somme des composants dans la composition valant 100% en poids.

11. Composition selon la revendication 10, présentant une teneur en 3-glycidyloxypropyltri(-2-éthylhexoxy)silane ou en 3-glycidyloxypropyltri(-2-propylheptoxy)silane ≥ 90% en poids, de préférence ≥ 95%.

12. Composition selon l'une quelconque des revendications 10 ou 11, présentant une teneur en alcools, tels que le méthanol ou l'éthanol, ≤ 1% en poids, de préférence ≤ 0,5% en poids, par rapport à la composition.

13. Composition selon l'une quelconque des revendications précédentes, présentant une teneur en esters mixtes ≤ 10% en poids, de préférence ≤ 5% en poids, ces esters mixtes étant de préférence choisis dans la série formée par le 3-glycidyloxypropyldi(-2-éthylhexoxy)monométhoxysilane, le 3-glycidyloxypropyldi(-2-éthylhexoxy)monoéthoxysilane, le 3-glycidyloxypropylmono(-2-éthylhexoxy)diméthoxysilane ainsi que le 3-glycidyloxypropylmono(-2-éthylhexoxy)diéthoxysilane.

14. Utilisation d'au moins un 3-glycidyloxypropyltrialcoxysilane présentant des groupes alcoxy à longue chaîne selon l'une quelconque des revendications précédentes pour la fonctionnalisation de caoutchouc, du 3-glycidyloxypropyltri(-2-éthylhexoxy)silane ou du 3-glycidyloxypropyltri(-2-propylheptoxy)silane étant de préférence utilisé pour l'interruption des chaînes lors d'une polymérisation par voie anionique.

15. Utilisation d'au moins un 3-glycidyloxypropyltrialcoxysilane présentant des groupes alcoxy à longue chaîne selon l'une quelconque des revendications précédentes pour la modification de caoutchouc.

16. Utilisation d'un caoutchouc modifié par au moins un 3-glycidyloxypropyltrialcoxysilane présentant des groupes alcoxy à longue chaîne, de préférence le 3-glycidyloxypropyltri(-2-éthylhexoxy)silane ou le 3-glycidyloxypropyltri(-2-propylheptoxy)silane selon l'une quelconque des revendications précédentes dans des mélanges de caoutchouc.

17. Utilisation d'un caoutchouc S-SBR ou BR modifié par du 3-glycidyloxypropyltri(-2-éthylhexoxy)silane ou du 3-glycidyloxypropyltri(-2-propylheptoxy)silane dans des mélanges de caoutchouc selon l'une quelconque des revendications précédentes pour des bandes de roulement dans des pneus.
